(19) Europäisches Patentamt
European Patent Office
Office européen des brevets

(11) Veröffentlichungsnummer: **0 155 574**
**B1**

(12) # EUROPÄISCHE PATENTSCHRIFT

(45) Veröffentlichungstag der Patentschrift: **24.08.88**

(51) Int. Cl.⁴: **C 07 D 405/12**, A 01 N 43/40 // C07D307/91

(21) Anmeldenummer: **85102326.7**

(22) Anmeldetag: **01.03.85**

(54) **Pyridiniumsalze und diese enthaltende Fungizide und Bakterizide.**

(30) Priorität: **10.03.84 DE 3408879**

(43) Veröffentlichungstag der Anmeldung:
**25.09.85 Patentblatt 85/39**

(45) Bekanntmachung des Hinweises auf die Patenterteilung:
**24.08.88 Patentblatt 88/34**

(84) Benannte Vertragsstaaten:
**BE CH DE FR GB LI NL**

(56) Entgegenhaltungen:
**EP - A - 0 057 362**
**EP - A - 0 103 277**
**US - A - 2 578 668**

**COLLECTION CZECHOSLOVAK CHEMICAL COMMUNICATIONS, Band 48, Nr. 7, Juli 1983, Seiten 1891-1897, Prag, CS; DANIEL VEGH u.a.: "Synthesis of pyridinium and picolinium salts of 5-nitro-2-vinylfuran having antibacterial properties"**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Rentzea, Costin, Dr., Richard-Kuhn-Strasse 1-3, D-6900 Heidelberg (DE)**
Erfinder: **Sauter, Hubert, Dr., Neckarpromenade 20, D-6800 Mannheim 1 (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-67900 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft neue Pyridiniumsalze, Verfahren zu ihrer Herstellung und fungizide und bakterizide Mittel, die diese Verbindungen als Wirkstoffe enthalten.

Es ist bekannt, N-Trichlormethylthiotetrahydrophthalimid oder N-Trichlormethylthiophthalimid als Fungizide zu verwenden (Chem. Week, June 21, Seite 46 und Seite 63, 1972). Es ist ferner bekannt, $C_{12}/C_{14}$-Alkyldimethyl-benzyl-ammoniumchlorid zur Bekämpfung von Algen zu verwenden (Wallhäuser, Sterilisation – Desinfektion – Konservierung. Georg Thieme Verlag. Stuttgart 1978, S. 359). Auch quartäre Pyridiniumhalogenide mit fungizider Wirkung, die Dicyclohexylreste enthalten, sowie Dibenzofuranderivate mit fungizider Wirkung, die Piperidiniumreste enthalten, sind bekannt (US-2 578 668, EP-57 362). Quartäre Ammoniumsalze von Dibenzofuranderivaten mit fungizider Wirkung sind Gegenstand einer älteren Patentanmeldung (EP-103 277). Es wurde gefunden, daß Pyridiniumsalze der Formel

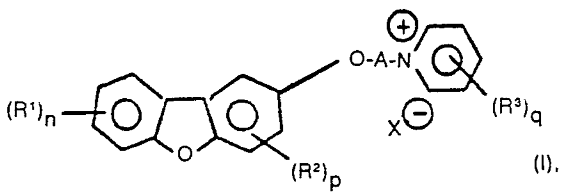

(I),

in der $R^1$ und $R^2$ gleich oder verschieden sind und Halogen, eine gegebenenfalls halogensubstituierte Alkyl- oder Alkoxygruppe mit 1 bis 4 Kohlenstoffatomen, Cyan oder Nitro, $R^3$ Halogen, einen Alkyl-, Alkenyl- oder Alkinylrest jeweils mit bis zu 6 Kohlenstoffatomen, Cyan, Nitro oder die Gruppe $-COOR^4$, $-CONR^4R^5$ oder $-NR^4R^5$, in der $R^4$ und $R^5$ gleich oder verschieden sind und unabhängig voneinander Wasserstoff oder einen Alkylrest mit 1 bis 6 Kohlenstoffatomen, n, p und q die Zahlen 0, 1, 2 oder 3, A eine gegebenenfalls durch eine oder mehrere Alkylgruppen mit jeweils 1 bis 3 C-Atomen substituierte Alkylenkette mit 2 bis 10 C-Atomen bedeuten und X das Anion einer nicht phytotoxischen Säure HX bedeutet, fungizid, bakterizid und algizid gut wirksam sind.

Als Bedeutung für $R^1$ und $R^2$ in Formel I kommen Halogen, wie Fluor, Chlor, Brom und Jod, Nitro, Cyano oder gegebenenfalls durch Halogen substituierte Alkyl- oder Alkoxyreste mit 1 bis 4 Kohlenstoffatomen, wie Methyl, Ethyl, Isopropyl, tert-Butyl, Methoxy, Ethoxy, Trifluormethyl, Difluormethyl, Tetrafluorethoxy, in Betracht.

$R^3$ bedeutet beispielsweise Fluor, Chlor, Brom, einen Alkyl-, Alkenyl- oder Alkinylrest, jeweils mit bis zu 6 Kohlenstoffatomen, wie Methyl, Ethyl, n-Propyl, Isopropyl, n-Butyl, sek.-Butyl, iso-Butyl, Pentyl, Allyl, Propargyl, ferner Cyan und Nitro. $R^4$ und $R^5$ bedeuten beispielsweise Wasserstoff, Methyl, Ethyl, n-Propyl, n-Butyl, iso-Butyl oder n-Pentyl, n und q bedeuten die Zahlen 0, 1, 2 und 3 und p die Zahl 0.

X in Formel I bedeutet das Anion einer einbasigen, nicht phytotoxischen Säure oder ein Äquivalent einer mehrbasigen, nicht phytotoxischen Säure, beispielsweise von Chlorid, Bromid, Hydrogensulfat, Phenylsulfonat, p-Methylphenylsulfonat oder ein Äquivalent des Sulfat-Anions.

Verbindungen der Formel (I), worin $R^1$ Halogen, Methyl, Trifluormethyl oder Nitro, $R^3$ Methyl, Ethyl n-Propyl, n-Butyl, iso-Butyl, Chlor, Cyan oder $-NR^4R^5$, $R^4$ und $R^5$ Wasserstoff, Methyl, Ethyl oder n-Propyl, n und q die Zahlen 0, 1, 2 und 3 und p die Zahl 0, A eine gegebenenfalls durch eine Alkylgruppe mit 1 bis 3 C-Atomen substituierte Alkylenkette mit 2 bis 10 C-Atomen und X das Anion einer nicht phytotoxischen Säure HX bedeutet, sind bevorzugt.

Man erhält die Pyridiniumsalze der Formel I durch Umsetzung von Verbindungen der Formel

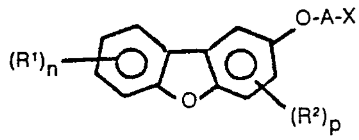

(II),

in der $R^1$, $R^2$, A, X, n und p die oben angegebenen Bedeutungen haben, mit einem Pyridin-Derivat der Formel

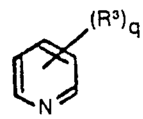

(III),

in der $R^3$ und q die oben angegebenen Bedeutungen haben. Die Reaktion wird gegebenenfalls in Gegenwart eines Lösungs- oder Verdünnungsmittels bei einer Temperatur im Bereich zwischen 20 und 150 °C, vorzugsweise zwischen 30 und 140 °C, durchgeführt. Der Ausgangsstoff der Formel II wird zweckmäßig mit dem 2- bis 10fachen molaren Überschuß des Pyridins der Formel III umgesetzt.

Als bevorzugte, gegenüber den Reaktionsteilnehmern inerte Lösungs- oder Verdünnungsmittel können beispielsweise aliphatische oder aromatische, gegebenenfalls halogenierte Kohlenwasserstoffe, wie Pentan, Cyclohexan, Benzol, Toluol, Xylol, Chlorbenzol, Dichlorbenzole, aliphatische Ketone wie Aceton, Methylethylketon, Diethylketon oder Cyclopentanon, Ether wie Diethylether, Dimethoxyethan, Tetrahydrofuran oder Dioxan, Ester, wie Essigsäureethylester, Nitrile wie Acetonitril, Amide wie Dimethylformamid, Dimethylacetamid und N-Methylpyrrolidon oder Gemische dieser Lösungsmittel verwendet werden.

Die Ausgangsstoffe der Formel II können leicht nach bekannten Verfahren hergestellt werden, beispielsweise durch Veretherung von Dibenzofuran-3-olen mit aliphatischen Dihalogeniden, bevorzugt in siedendem Aceton, Methylethylketon, Diethylketon oder Cyclopentanon oder in Dimethylformamid in Gegenwart von mindestens äquivalenten Mengen Natrium- oder Kaliumcarbonat (Houben-Weyl-Müller, Methoden der Organischen Chemie, Band 6/3, Seiten 54 bis 59, Georg Thieme Verlag, Stuttgart, 1965).

Alternativ können Dibenzofuran-3-oxyalkanole mit Thionylchlorid oder Phosphortribromid zu Verbindungen der Formel II umgesetzt werden (Rec.

trav. chim., 76, 129 bis 146 [1957]). Dibenzofuran-3-oxyalkanole erhält man durch Umsetzung von Dibenzofuran-3-olen mit aliphatischen Oxiranen oder aliphatischen, cyclischen Carbonaten nach bekannten Methoden. Weiterhin lassen sich entsprechend Literaturangaben (J. Med. Chem., 17, Seite 108 [1974]) hergestellte alpha-(Dibenzofuran-3-oxy)-carbonsäureester katalytisch oder mit komplexen Hydriden zu Dibenzofuran-3-oxyalkanolen reduzieren.

Als gegebenenfalls alkylsubstituierte Alkylenketten A kommen beispielsweise in Betracht $-CH(CH_3)-CH_2-$, $-CH_2-CH(CH_3)-$, $-CH(C_2H_5)-CH_2$, $-CH_2-CH_2-$, $-(CH_2)_3-$, $-(CH_2)_4-$, $-CH(CH_3)CH_2CH_2CH_2-$, $-(CH_2)_5-$, $-(CH_2)_6-$, $-(CH_2)_7-$, $-(CH_2)_8-$.

Für die Herstellung von Ausgangsverbindungen der Formel II können beispielsweise die folgenden Dibenzofuran-3-ole eingesetzt werden: Dibenzofuran-3-ol, 7-Fluor-dibenzofuran-3-ol, 5-Chlor-dibenzofuran-3-ol, 6-Chlor-dibenzofuran-3-ol, 7-Chlor-dibenzofuran-3-ol, 8-Chlor-dibenzofuran-3-ol, 5,7-Dichlor-dibenzofuran-3-ol, 6,7-Dichlor-dibenzofuran-3-ol; 7,8-Dichlor-dibenzofuran-3-ol, 2,4,7-Trichlor-dibenzofuran-3-ol, 7-Brom-dibenzofuran-3-ol, 7-Methyl-dibenzofuran-3-ol, 7-Methoxy-dibenzofuran-3-ol, 6,8-Dichlor-7-methyl-dibenzofuran-3-ol, 6-Trifluormethyl-dibenzofuran-3-ol, 7-Trifluormethyl-dibenzofuran-3-ol, 7-Chlor-2-nitro-dibenzofuran-3-ol und 7-Ethoxy-dibenzofuran-3-ol.

Als Pyridine der Formel III kommen beispielsweise Pyridin, 2-, 3- und 4-Methylpyridin, 2-, 3- und 4-Ethylpyridin, 2-, 3- und 4-n-Propylpyridin, 2-, 3- und 4-n-Butylpyridin, 2-, 3- und 4-iso-Butylpyridin, 2-, 3- und 4-n-Pentylpyridin, 2,3-, 2,4-, 2,5-, 3,4- und 3,5-Dimethylpyridin, 2,4,6-Trimethylpyridin, 2-, 3- und 4-Allylpyridin, 2-, 3- und 4-Fluorpyridin, 2-, 3- und 4-Chlorpyridin, 2-, 3- und 4-Brompyridin, 2-, 3- und 4-Cyanpyridin, 2-, 3- und 4-Aminopyridin, 2-, 3- und 4-Dimethylaminopyridin, 2-, 3- und 4-Diethylaminopyridin, 2-, 3- und 4-Propylaminopyridin, 2-, 3- und 4-Butylaminopyridin, 2-, 3- und 4-Pyridincarbonsäure, 2-, 3- und 4-Pyridincarboxamid, 2-, 3- und 4-N,N(Dimethyl)-pyridincarboxamid und N,N(Diethyl)-pyridincarboxamid in Betracht.

Die folgenden Beispiele erläutern die Herstellung der Verbindungen der Formel I.

Beispiel 1

a) Zu einer Lösung von 98,4 g (0,45 Mol) 7-Chlor-dibenzofuran-3-ol in 15 ml trockenem Dimethylformamid werden 93 g (0.674 Mol) fein gepulvertes Kaliumcarbonat und 286 g (2 Mol) 1,6-Dichlorhexan zugegeben. Nach zehnstündigem Rühren bei 100 °C wird das Reaktionsgemisch auf 20 °C abgekühlt und der anorganische Niederschlag abgesaugt. Das Filtrat wird unter vermindertem Druck eingeengt. Der Rückstand wird in 2 l Diethylether aufgenommen, dreimal mit Wasser gewaschen, getrocknet und mit Tierkohle entfärbt. Nach Einengen der organischen Phase erhält man 85.2 g 1-(7'-Chlor-3'-dibenzofuranoxy)-6-chlorhexan vom Schmp. 73–75 °C.

b) 12 g (0.0356 Mol) 1-(7'-Chlor-3'-dibenzofuranoxy)-6-chlorhexan gelöst in 30 ml trockenem Dimethylformamid, werden mit 25 ml 3-Methylpyridin versetzt, 6 Stunden bei 100 °C gerührt und anschließend unter vermindertem Druck eingeengt. Der Rückstand wird 1 Stunde mit 200 ml trockenem Ether bei + 10 °C gerührt, und der weiße, kristalline Niederschlag wird abgesaugt, mit Ether nachgewaschen und getrocknet. Man erhält 11.4 g 1-(7'-Chlor-3'-dibenzofuranoxy)-6-(3'-methylpyridinium) hexanchlorid vom Schmp. 188–190 °C (Verbindung Nr. 1).

Entsprechend Beispiel 1 lassen sich beispielsweise folgende Verbindungen der Formel I herstellen:

| Verb.-Nr. | $R^1_n$ | A | p | $R^3_q$ | X | Fp (°C) |
|---|---|---|---|---|---|---|
| 2 | H | $-CH(CH_3)CH_2-$ | O | $3-NH-CH_2CH_2CH_3$ | Br | Harz |
| 3 | H | $-(CH_2)_5-$ | O | $3-CH_2CH_2CH_2CH_3$ | Br | Harz |
| 4 | 6-Chlor | $-CH(CH_3)CH_2-$ | O | $3-COOCH_2CH_2CH_2CH_3$ | Br | Harz |
| 5 | 6-Chlor | $-(CH_2)_2-$ | O | $3-CH_3$ | Br | 183–185 |
| 6 | 6-Chlor | $-(CH_2)_{10}-$ | O | $3-CON(CH_2CH_2CH_3)_2$ | Br | Harz |
| 7 | 7-Chlor | $-(CH_2)_2-$ | O | $3-CH_3$ | Cl | 217–218 |
| 8 | 7-Chlor | $-(CH_2)_3-$ | O | $4-CH_3$ | Br | 168–170 |
| 9 | 7-Chlor | $-(CH_2)_3-$ | O | $3-CH_3$ | Br | 119–120 |
| 10 | 7-Chlor | $-CH(C_2H_5)CH_2CH_2$ | O | $3-CH_2CH_2CH_2CH_3$ | Br | Harz |
| 11 | 7-Chlor | $-(CH_2)_3-$ | O | $3-C_2H_5$ | Br | 117–119 |
| 12 | 7-Chlor | $-(CH_2)_4-$ | O | $3-CH_3$ | Br | 129–133 |
| 13 | 7-Chlor | $-(CH_2)_4-$ | O | $3-C_2H_5$ | Br | 112–113 |
| 14 | 7-Chlor | $-(CH_2)_4-$ | O | $3-CH_2CH_2CH_2CH_3$ | Br | 83–85 |
| 15 | 7-Chlor | $-(CH_2)_4-$ | O | 3-CN | Br | 224–226 |
| 16 | 7-Chlor | $-(CH_2)_6-$ | O | H | Br | 133–135 |
| 17 | 7-Chlor | $-(CH_2)_6-$ | O | $4-CH_3$ | Br | 116–119 |
| 18 | 7-Chlor | $-(CH_2)_6-$ | O | $3-CH_3$ | Br | 173–176 |
| 19 | 7-Chlor | $-(CH_2)_6-$ | O | $3-C_2H_5$ | Br | 127–128 |
| 20 | 7-Chlor | $-(CH_2)_7-$ | O | $3-CH_3$ | Br | 129–130 |
| 21 | 7-Chlor | $-(CH_2)_7-$ | O | $4-CH_3$ | Br | 138–140 |
| 22 | 7-Chlor | $-(CH_2)_8-$ | O | $3-CH_3$ | Br | 156–158 |
| 23 | 7-Chlor | $-(CH_2)_8-$ | O | $4-CH_3$ | Br | 106–109 |

| Verb.-Nr. | $R_n^1$ | A | p | $R_q^3$ | X | Fp (°C) |
|---|---|---|---|---|---|---|
| 24 | 7-Chlor | $-(CH_2)_8-$ | O | $4-N(CH_3)_2$ | Br | 170–172 |
| 25 | 7-Chlor | $-(CH_2)_6-$ | O | $4-N(CH_3)_2$ | Br | 181–183 |
| 26 | 7-Chlor | $-(CH_2)_{10}-$ | O | 3-Br | Br | Harz |
| 27 | 7-Fluor | $-(CH_2)_6-$ | O | $4-CH_3$ | Br | 130–132 |
| 28 | 7-Brom | $-(CH_2)_6-$ | O | $4-CH_3$ | Br | 99–102 |
| 29 | 7-Brom | $-(CH_2)_6-$ | O | 3,5-Dimethyl | Br | 163–166 |
| 30 | 7-Brom | $-(CH_2)_{10}-$ | O | 2,4,6-Trimethyl | Br | Harz |
| 31 | $6-CF_3$ | $-(CH_2)_2-$ | O | $3-CH_3$ | Br | 182–183 |
| 32 | $6-CF_3$ | $-(CH_2)_6-$ | O | $4-CH_3$ | Br | 60– 62 |
| 33 | $6-CF_3$ | $-(CH_2)_6-$ | O | $3-CH_3$ | Br | 140–142 |
| 34 | $6-CF_3$ | $-(CH_2)_6-$ | O | $3-C_2H_5$ | Br | 141–143 |
| 35 | $6-CF_3$ | $-(CH_2)_6-$ | O | 3-Cl | Br | 223–225 |
| 36 | $7-CF_3$ | $-(CH_2)_8-$ | O | $4-CO-N(CH_2CH_2CH_3)_2$ | Br | Harz |
| 37 | $7-CH_3$ | $-(CH_2)_6-$ | O | $2-COOCH_2CH_2CH_2CH_3$ | Br | Harz |
| 38 | $6.7-Cl_2$ | $-(CH_2)_6-$ | O | $4-CH_3$ | Br | 152–155 |
| 39 | $6,7-Cl_2$ | $-(CH_2)_6-$ | O | $3-CH_3$ | Br | 191–192 |
| 40 | $6,8-Cl_2$, $7-CH_3$ | $-(CH_2)_6-$ | O | $4-CH_3$ | Br | 112–114 |
| 41 | $6.8-Cl_2$. $7-CH_3$ | $-(CH_2)_6-$ | O | $3-CH_3$ | Br | 107–110 |

Die neuen Wirkstoffe zeigen eine starke fungitoxische Wirksamkeit gegen phytopathogene Pilze. Sie dienen insbesondere zur Verhütung und Heilung von Pflanzenkrankheiten, die durch Mikroorganismen verursacht werden, wie z.B. Botrytis cinerea, Plasmopara viticola, Monilia fructigena, Alternaria solani, Sclerotinia sclerotiorum, Pyricularia oryzae, Pellicularia sasakii, Erysiphe graminis, Erysiphe cichoracearum, Chaetomium globosum, Aspergillus niger, Xanthomonas oryzae, Xanthomonas citri, Phytophthora infestans (an Kartoffeln und Tomaten). Besonders gut geeignet ist die Verbindung Nr. 17. Die Wirkstoffe eignen sich ferner für die Bekämpfung von Algen.

Die neuen Wirkstoffe können gleichzeitig das Wachstum von zwei oder mehr der genannten Pilze unterdrücken und besitzen eine hohe Pflanzenverträglichkeit. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen.

Die fungiziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 bis 5 kg Wirkstoff je ha.

Die neuen Wirkstoffe können auch zusammen mit anderen Wirkstoffen, z.B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten. Eine besonders günstige Vergrößerung des Wirkungsspektrums wird mit folgenden Fungiziden erzielt:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester
heterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-[dimethylamino]-phosphinyl-3-phenyl-1,2,4-triazol)
2,3-Dicyano-1,4-dithiaanthrachion
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-[2]-benzimidazol)
2-(Thiazolyl-[4]-benzimidazol)
N-(1,1,2,2-Tetrachlorethylthio)-tetrahydrophthalimid
N-Trichlormethyl-phthalimid

N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexyl-amid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-[2,2,2-trichlor-ethyl])-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-(3-[p-tert.-Butylphenyl]-2-methylpropyl)-cis-2,6-dimethylmorpholin
N-(3-[p-tert.-Butylphenyl]-2-methylpropyl)-piperidin
1-(2-[2,4-Dichlorphenyl]-4-ethyl-1,3-dioxolan-2-yl-ethyl)-1H-1,2,4-triazol
1-(2-[2,4-Dichlorphenyl]-4-n-propyl-1,3-dioxolan-2-yl-ethyl)-1H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-[3,5-Dimethyl-2-oxycyclohexyl]-2-hydroxyethyl)-glutarimid
Hexachlorbenzol
DL-Methyl-N-(2,6-dimethyl-phenyl)-N-furoyl(2)-alaninat
DL-N-(2,6-Dimethyl-phenyl)-N-(2'-methoxyacetyl)-alanin-methylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion
3-(3,5-Dichlorphenyl)-1-isopropylcarbamoyl-hydantoin
N-(3,5-Dichlorphenyl)-1,2-dimethylcyclopropan-1,2-dicarbonsäureimid
2-Cyano-N-(ethylaminocarbonyl)-2-methoximino-acetamid
1-(2-[2,4-Dichlorphenyl]-pentyl)-1H-1,2,4-triazol
2,4-Difluor-alpha-(1H-1,2,4-triazolyl-1-methyl)-benzhydrylalkohol
Imidazolidinylharnstoff
Brom-nitrostyrol
Dimethoxy-tetrahydrofuran
6-Acetoxi-2,4-dimethyl-m-dioxan
1,3-Dimethylol-5,5-dimethylhydantoin
Hexahydro-1,3,5-tris-(2-oxiethyl)s-triazin
Benzalkoniumchlorid
Cetylpyridiniumchlorid
Alkyldimethyl-benzylammoniumchlorid
Mono- und Diguanidinderivate

Die neuen Wirkstoffe werden beispielsweise in Form von direkt versprühbaren Lösungen, Suspensionen, Emulsionen, auch in Form von hochprozentigen wäßrigen, öligen oder sonstigen Dispersionen, Pasten, Stäubemitteln, Streumitteln, Granulaten durch Versprühung, Verstäuben, Verstreuen, Verstreichen oder Gießen ausgebracht. Die Anwendungsformen richten sich ganz nach den Verwendungszwecken; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate z.B. Methanol, Ethanol, Propanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser in Betracht. Bevorzugt wird die Lösung in Wasser.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Phospolipide, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiele für solche Pflanzenschutzmittel-Zubereitungen sind:

I. Man vermischt 90 Gewichtsteile der Verbindung 7 mit 100 Gewichtsteilen N-Methylpyrrolidon und erhält eine Lösung, die zur Anwendung in Form kleinster Tropfen geeignet ist.

20 Gewichtsteile der Verbindung 5 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung 9 werden in einer Mischung gelöst, die aus 30 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und Verrühren der Lösung in Wasser erhält man eine wäßrige Dispersion.

IV. 20 Gewichtsteile der Verbindung 17 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und Verrühren der Lösung in Wasser erhält man eine wäßrige Dispersion.

V. 20 Gewichtsteile der Verbindung 9 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der Verbindung 14 werden mit 95 Gewichtsteilen feinteiligem Kaolin innig vermischt. Man erhält auf diese Weise ein Stäubemittel, das 5 Gew.-% des Wirkstoffs enthält.

VII. 30 Gewichtsteile der Verbindung 11 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung 15 werden mit 30 Teilen Natriumsalz eines Phenolsulfonsäure-Harnstoff-Formaldehyd-Kondensates, 2 Teilen Kieselgel und 48 Teilen Wasser innig vermischt. Man erhält eine stabile wäßrige Dispersion.

IX. 20 Teile der Verbindung 17 werden mit 2 Teilen Calciumsalz der Dedecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Auch für technische Zwecke im Materialschutz, z.B. als Holzschutzmittel, sind die neuen Ammoniumsalze geeignet. Über die fungizide Wirkung hinaus wurde auch eine bakterizide Wirksamkeit der Verbindungen und eine Wirkung gegen Algen festgestellt, so daß sie auch als solche im Pflanzenschutz- und als technische Mikrozide in Betracht kommen. Beispielsweise können folgende Mikroorganismen damit bekämpft werden:

Staphylococcus aureus, Escherichia coli, Klebsiella pneumoniae, Citrobacter freundii, Proteus vulgaris, Pseudomonas aeruginosa, Pseudomonas fluorescens, Xanthomonas vesicatoria, Xanthomonas malvaccanium, Erwinia carotovora, Erwinia amylovora, Desulfovibrio desulfuricans, Streptoverticillium rubrireticuli, Aspergillus niger, Aspergillus versicolor, Penicillium funiculosum, Paecilomyces variotii, Trichoderma viride, Chaetomium globosum, Candida albicans, Geotrichum candidans, Monilia sitophila, Scenedesmus quadricauda, Chlorella vulgaris, Nostoc muscorum.

Die Verbindungen eignen sich auch zur Schimmelpilzbekämpfung in Feuchträumen, auf Tapeten, Holzgegenständen, Textilien und Leder. Ferner können sie zur äußeren Anwendung gegen Pilze in der Human- und Veterinärmedizin eingesetzt werden.

Wegen ihrer geringen Neigung zur Schaumbildung können sie auch als Bestandteil in Reinigungsmitteln mit desinfizierender Wirksamkeit verwendet werden.

Die üblichen Anwendungskonzentrationen betragen 0,01 bis 1% Wirkstoff, bezogen auf das Gewicht des zu schützenden Materials; beim Einsatz zur Wasserbehandlung bei der Erdölförderung, in Schwimmbädern, Rückkühlwerken, Luftbefeuchtungsanlagen, Blumenfrischhaltemitteln oder in der Papierindustrie sind Wirkstoffmengen von 5 bis 100 ppm ausreichend, um eine Mikroorganismenentwicklung zu unterdrücken. Gebrauchsfertige Desinfektionsmittellösungen enthalten 0,2 bis 5% Wirkstoff.

Die folgenden Versuche erläutern die biologische Wirkung der neuen Verbindungen. Als Vergleichsmittel wurden die bekannten Wirkstoffe N-Trichlormethylthio-tetrahydrophthalimid (A), N-Trichlormethylthiophthalimid (B) und N–$C_{12}$/$C_{14}$-Alkyl-N,N-dimethyl-N-benzylammoniumchlorid (C) verwendet.

Beispiel 1

Wirksamkeit gegen Phytophthora infestans an Tomaten

Blätter von Topfpflanzen der Sorte «Große Fleischtomate» werden mit wäßriger Spritzbrühe, die 80% Wirkstoff und 20% Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18 °C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß beispielsweise bei der Anwendung als 0,025%ige Wirkstoffbrühe die Wirkstoffe 5, 7, 9, 11, 14, 15, 17, 18, 19, 22, 27, 33, 34 und 39 eine gute fungizide Wirkung zeigten (beispielsweise 97%) z.T. bei leichtem Blattschaden.

Beispiel 2

Wirksamkeit gegen Botrytis cinerea an Paprika

Paprikasämlinge der Sorte «Neusiedler Ideal Elite» werden, nachdem sich 4 bis 5 Blätter gut entwickelt haben, mit wäßrigen Suspensionen, die 80% Wirkstoff und 20% Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise bei der Anwendung als 0,05%ige Wirkstoffbrühe die Wirkstoffe 1, 8, 13, 14, 15, 16, 19, 22, 27, 29, 35 und 39 eine bessere fungizide Wirkung zeigten (beispielsweise 97%) als der bekannte Wirkstoff A (beispielsweise 70%).

Beispiel 3

Wirksamkeit gegen Plasmopara viticola

Blätter von Topfreben der Sorte «Müller-Thurgau» werden mit wäßriger Spritzbrühe, die 80% Wirkstoff und 20% Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24 °C und anschließend für 8 Tage in einem Gewächshaus mit Temperaturen zwischen 20 und 30 °C aufgestellt. Nach dieser Zeit werden die Pflanzen zur Beschleunigung des Sporangienträgerausbruches abermals für 16 Stunden in der feuchten Kammer aufgestellt.

Dann erfolgt die Beurteilung des Ausmaßes des Pilzausbruches auf den Blattunterseiten.

Das Ergebnis des Versuches zeigt, daß beispielsweise bei der Anwendung als 0,05%ige Wirkstoffbrühe die Wirkstoffe 1, 7, 20, 22 und 32 eine bessere fungizide Wirkung zeigten (beispielsweise 97%) als der bekannte Wirkstoff B (beispielsweise 90%).

Beispiel 4

Zur Ermittlung der Wirksamkeit der neuen Verbindungen gegenüber Bakterien werden zu je 5 ml steigender Verdünnung der Wirkstoffe 5 ml doppelt konzentrierter Nährbouillon in sterile Reagenzgläser gegeben und vermischt. Durch Zugabe von einem Tropfen einer 1:10 verdünnten 16 Stunden alten Bouillon-Kultur der Bakterien-Arten Staphylococcus aureus bzw. Escherichia coli oder Proteus vulgaris werden dann die Röhrchen beimpft und 24 Stunden bei 37 °C bebrütet. Nach dieser Zeit werden Proben aus den Röhrchen auf Bakteriennährböden übertragen und diese ebenfalls 24 Stunden lang bei 37 °C bebrütet. Diejenige Verdünnungsstufe, bei welcher nach dem Übertragen einer Probe auf den Nährboden keine Bakterienentwicklung mehr erfolgt, wird als Abtötungswert angegeben.

Das Ergebnis des Versuches zeigt, daß die Wirkstoffe 17 und 18 eine gute bakterizide Wirkung zeigen (beispielsweise Abtötungswert 12 Teile Wirkstoff pro Million Teile Nährbouillon).

Beispiel 5

Zur Prüfung der Wirksamkeit gegenüber Pilzen werden die Wirkstoffe einer für das Wachstum der Pilze Aspergillus niger, Oidium lactis bzw. Candida albicans in Mengen von 100, 50, 25, 12, 6 und 3 Gewichtsteilen pro Million Teile Nährlösung zugesetzt. Jeweils 10 ml Nährlösungs-Wirkstoffgemisch werden in sterile Reagenzgläser gegeben und mit einem Tropfen einer Sporensuspension beimpft, die $10^6$ Konidien bzw. Zellen enthält. Nach 120stündiger Bebrütung werden aus denjenigen Röhrchen, die kein sichtbares Pilzwachstum zeigen, Proben entnommen und auf Pilznährböden übertragen. Es wird die Verdünnungsstufe ermittelt, bei welcher nach dem

Übertragen einer Probe auf den Nährboden kein Wachstum der Pilze mehr erfolgt.

Das Ergebnis des Versuches zeigt, daß die Wirkstoffe 17 und 18 eine gute fungizide Wirkung haben (beispielsweise Verdünnung 12 Teile Wirkstoff pro Million Teile Nährlösung).

Beispiel 6

Zur Prüfung der Wirksamkeit gegenüber Grünalgen werden die Wirkstoffe einer für die Vermehrung der einzelligen Grünalge Chlorella vulgaris begünstigenden, phosphatreichen Nährlösung in Mengen von 10, 5 und 1 Gewichtsteilen pro Million Teile Nährlösung zugesetzt. Jeweils 100 ml Nährlösungs-Wirkstoffgemisch sowie nur Nährlösung (Kontrollen) werden in 300-ml-Glaskolben gegeben. Die Nährlösung wird vor dem Wirkstoffzusatz mit einer Suspension der Alge Chlorella vulgaris beimpft; die Zelldichte wird auf $10^6$ Zellen/ml Nährlösung eingestellt. Nach 20tägiger Aufstellung der Versuchsansätze bei Raumtemperatur und Lichtzutritt wird die Wirksamkeit beurteilt.

Das Ergebnis des Versuches zeigt, daß die Wirkstoffe 7, 13 und 18 eine bessere Wirkung gegen Algen haben (beispielsweise 1 Teil Wirkstoff pro Million Teile Nährlösung) als der bekannte Wirkstoff C (beispielsweise 10 Teile Wirkstoff pro Million Teile Nährlösung).

**Patentansprüche**

1. Pyridiniumsalz der Formel

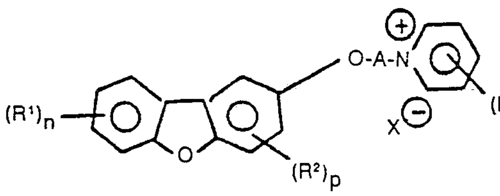

(I)

in der

$R^1$ und $R^2$ gleich oder verschieden sind und Halogen, eine gegebenenfalls halogensubstituierte Alkyl- oder Alkoxygruppe mit 1 bis 4 Kohlenstoffatomen, Cyan oder Nitro, $R^3$ Halogen, einen Alkyl-, Alkenyl- oder Alkinylrest jeweils mit bis zu 6 Kohlenstoffatomen, Cyan, Nitro oder die Gruppe $-COOR^4$, $CO-NR^4R^5$, oder $-NR^4R^5$, in der $R^4$ und $R^5$ gleich oder verschieden sind und unabhängig voneinander Wasserstoff oder einen Alkylrest mit 1 bis 6 Kohlenstoffatomen,

n, p und q die Zahlen 0, 1, 2 oder 3,

A eine gegebenenfalls durch eine oder mehrere Alkylgruppen mit jeweils 1 bis 3 C-Atomen substituierte Alkylenkette mit 2 bis 10 C-Atomen bedeuten und

X das Anion einer nicht phytotoxischen Säure HX bedeutet.

2. Pyridiniumsalz der Formel I gemäß Anspruch 1, dadurch gekennzeichnet, daß $R^1$ Halogen, Methyl, Trifluormethyl oder Nitro, $R^3$ Methyl, Ethyl, n-Propyl, n-Butyl, iso-Butyl, Chlor, Cyan oder $-NR^4R^5$, $R^4$ und $R^5$ Wasserstoff, Methyl, Ethyl oder n-Propyl, n und q die Zahlen 0, 1, 2 und 3 und p die Zahl 0, A eine gegebenenfalls durch eine Alkylgruppe mit 1 bis 3 C-Atomen substituierte Alkylenkette mit 2 bis 10 C-Atomen und X das Anion einer nicht phytotoxischen Säure HX bedeutet.

3. Verfahren zur Herstellung von Pyridiniumsalzen der Formel I gemäß Anspruch 1, dadurch gekennzeichnet, daß man eine Verbindung der Formel

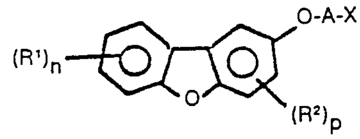

(II)

in der $R^1$, $R^2$, A, X, n und p die im Anspruch 1 angegebenen Bedeutungen haben, mit einem Pyridin der Formel

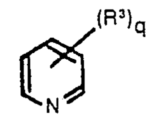

(III),

in der $R^3$ und q die im Anspruch 1 angegebenen Bedeutungen haben, umsetzt.

4. Fungizides und bakterizides Mittel, enthaltend ein Pyridiniumsalz der Formel I gemäß Anspruch 1.

5. Fungizides und bakterizides Mittel, enthaltend inerte Zusatzstoffe und ein Pyridiniumsalz der Formel I gemäß Anspruch 1.

6. Verfahren zur Bekämpfung von Pilzen und Bakterien, dadurch gekennzeichnet, daß man ein Pyridiniumsalz der Formel I gemäß Anspruch 1 auf die Pilze und/oder Bakterien oder auf durch Pilz- und oder Bakterienbefall bedrohte Materialien, Flächen, Pflanzen oder Saatgüter einwirken läßt.

7. Verfahren zur Herstellung eines fungiziden und bakteriziden Mittels, dadurch gekennzeichnet, daß man ein Pyridiniumsalz der Formel I gemäß Anspruch 1 mit einem festen oder flüssigen Trägerstoff sowie gegebenenfalls mit einem oder mehreren oberflächenaktiven Mitteln mischt.

**Claims**

1. A pyridinium salt of the formula

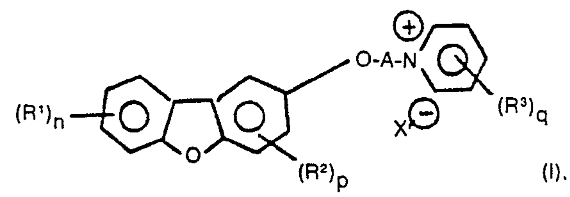

(I),

where $R^1$ and $R^2$ are identical or different and are each halogen, an unsubstituted or halogen-substituted alkyl or alkoxy group of 1 to 4 carbon atoms, cyano or nitro, $R^3$ is halogen, an alkyl, alkenyl or alkynyl radical, each of not more than 6 carbon atoms, cyano, nitro or a group $-COOR^4$, $CO-NR^4R^5$ or $-NR^4R^5$, where $R^4$ and $R^5$ are identical or different and independently of one another are each hydrogen or alkyl of 1 to 6 carbon atoms, n, p and q are each 0, 1, 2 or 3, A is an alkylene chain of 2 to 10 carbon atoms which is unsubstituted or substituted by one or more alkyl groups, each 1 to 3 carbon atoms, and X is an anion of a nonphytotoxic acid HX.

2. A pyridinium salt of the formula I as claimed in claim 1, wherein $R^1$ is halogen, methyl, trifluoromethyl or nitro. $R^3$ is methyl, ethyl, n-propyl, n-butyl, isobutyl, chlorine, cyano or $-NR^4R^5$, $R^4$ and $R^5$ are each hydrogen, methyl, ethyl or n-propyl, n and q are each 0, 1 or 2 or 3 and p is 0, A is an alkylene chain of 2 to 10 carbon atoms which is unsubstituted or substituted by an alkyl group of 1 to 3 carbon atoms, and X is an anion of a nonphytotoxic acid HX.

3. A process for the preparation of a pyridinium salt of the formula I as claimed in claim 1, wherein a compound of the formula

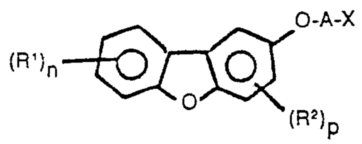

(II)

where $R^1$, $R^2$, A, X, n and p have the meanings stated in claim 1, is reacted with a pyridine of the formula

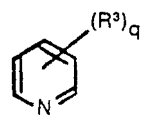

(III),

where $R^3$ and q have the meanings stated in claim 1.

4. A fungicide and bactericide containing a pyridinium salt of the formula I as claimed in claim 1.

5. A fungicide and bactericide containing inert additives and a pyridinium salt of the formula I as claimed in claim 1.

6. A method for controlling fungi and bacteria, wherein a pyridinium salt of the formula I as claimed in claim 1 is allowed to act on the fungi and/or bacteria or on materials, areas, plants or seeds threatened by fungal and/or bacterial attack.

7. A process for the preparation of a fungicide and bactericide, wherein a pyridinium salt of the formula I as claimed in claim 1 is mixed with a solid or liquid carrier and, if required, with one or more surfactants.

**Revendications**

1. Sel de pyridinium de formule

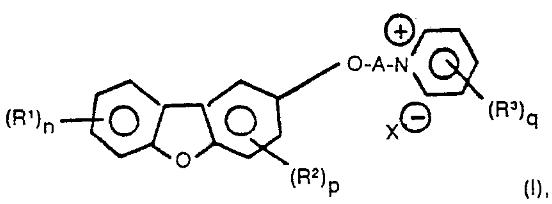

(I),

dans laquelle

$R^1$ et $R^2$ sont identiques ou différents et représentent halogène, un groupe alkyle ou alcoxy de 1 à 4 atomes de carbone, éventuellement substitué par halogène, cyano ou nitro.

$R^3$ représente halogène, un reste alkyle, alcényle ou alcinyle ayant chacun jusqu'à 6 atomes de carbone, cyano, nitro ou les groupes $-COOR^4$, $-CONR^4R^5$, ou $-NR^4R^5$ où $R^4$ et $R^5$ sont identiques ou différents et représentent, indépendamment l'un de l'autre, hydrogène ou un reste alkyle de 1 à 6 atomes de carbone,

n, p et q représentent les nombres 0, 1, 2 ou 3.

A représente une chaîne alkylène de 2 à 10 atomes C, éventuellement substituée par un ou plusieurs groupes alkyle ayant chacun 1 à 3 atomes C, et

X représente l'anion d'un acide HX non phytotoxique.

2. Sel de pyridinium de formule I selon la revendication 1, caractérisé par le fait que

$R^1$ représente halogène, méthyle, trifluorométhyle ou nitro, $R^3$ méthyle, éthyle, n-propyle, n-butyle, iso-butyle, chlore, cyano ou $-NR^4R^5$, $R^4$ et $R^5$ hydrogène, méthyle, éthyle ou n-propyle, n et q les nombres 0, 1, 2 et 3 et p le nombre 0.

A représente une chaîne alkylène de 2 à 10 atomes C éventuellement substituée par un groupe alkyle de 1 à 3 atomes C et X représente l'anion d'un acide HX non phytotoxique.

3. Procédé de préparation de sels de pyridinium de formule I selon la revendication 1, caractérisé par le fait que l'on fait réagir un composé de formule

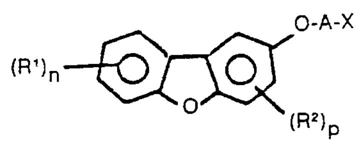

(II),

dans laquelle $R^1$, $R^2$, A, X, n et p ont les significations données dans la revendication 1, avec une pyridine de formule

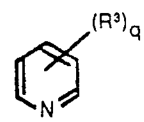

(III)

dans laquelle $R^3$ et q ont les significations données dans la revendication 1.

4. Agent fongicide et bactéricide contenant un sel de pyridinium de formule I selon la revendication 1.

5. Agent fongicide contenant des additifs inertes et un sel de pyridinium de formule I selon la revendication 1.

6. Procédé de lutte contre champignons et bactéries, caractérisé par le fait que l'on fait agir un sel de pyridinium de formule I selon la revendication 1 sur les champignons et/ou bactéries ou sur les matériaux, surfaces, plantes ou semis menacés d'une attaque par champignons et/ou bactéries.

7. Procédé de préparation d'un agent fongicide et bactéricide, caractérisé par le fait que l'on mélange un sel de pyridinium de formule I selon la revendication 1 avec un support solide ou liquide, ainsi qu'éventuellement un ou plusieurs agents tensioactifs.